# FASCICULE DE BREVET EUROPEEN

(11) **EP 1 194 175 B1**
(45) Date de publication et mention de la délivrance du brevet: **09.04.2003**
(21) Numéro de dépôt: 00922730.7
(22) Date de dépôt: 25.04.2000
(51) Int. Cl.: A61L 9/22, B01D 53/32

(54) **PROCEDE DE TRAITEMENT DE MILIEU GAZEUX CONTENANT DES PARTICULES CONTAMINANTES**
VERFAHREN ZUM BEHANDELN VON KONTAMINIERENDEN TEILCHEN ENTHALTENDEN GASFÖRMIGEN MEDIEN
METHOD FOR TREATING A GASEOUS MEDIUM CONTAINING CONTAMINATING PARTICLES

(30) Priorité: 27.04.1999 FR 9905320
(43) Date de publication de la demande: 10.04.2002
(73) Titulaire: Ectium BV, 1083 GV Amsterdam (NL)
(72) Inventeur: DREAN, Henri, Louis, F-75016 Paris (FR)
(74) Mandataire: Thinat, Michel
(86) Numéro de dépôt international: FR0001079
(87) Numéro de publication internationale: WO00064499

(56) Documents cités:
- US-A- 5 445 798
- US-A- 5 822 980

## Description

La présente invention concerne un procédé de traitement de milieu gazeux contenant des particules contaminantes du type micro-organismes, bactéries ou virus.

Ce procédé peut notamment être utilisé pour le traitement de l'air circulant dans des dispositifs de climatisation, par exemple dans les hôpitaux ou les trains, ou encore pour le traitement des atmosphères de conservation, par exemple celles des cuves réfrigérées des chambres froides ou des réfrigérateurs.

L'invention concerne également un dispositif de traitement de milieu gazeux contenant des particules contaminantes.

Les dispositifs de climatisation connus permettent de réguler la température de l'air qu'ils traitent mais ne comportent pas de moyen pour éliminer d'éventuelles particules contaminantes présentes dans celui-ci, ce qui peut s'avérer très préjudiciable à la santé des personnes respirant cet air climatisé.

Par ailleurs, les réfrigérateurs actuellement en service comportent essentiellement des cuves réfrigérées à une température positive et inférieure à 10°C pour améliorer la conservation des matières vivantes. Cependant, aucun dispositif n'est prévu pour traiter les atmosphères environnantes de ces matières en cours de maturation et de dégradation biologique.

Or, la maturation des matières vivantes génère des composés organiques volatils, comme l'éthylène, ainsi que des particules contaminantes du type micro-organisme, bactérie, levure, moisissure et virus. La dispersion aéroportée de ces composés organiques volatils et de ces particules contaminantes provoque des altérations microbiologiques qui accélèrent généralement l'action des enzymes et donc l'autocatalyse de ces métabolismes de maturation et la dégénérescence des matières vivantes en phase de maturation, lesquelles sont également influencées par la température, l'humidité relative, la vitesse de circulation de l'air et l'intensité lumineuse. Ces phénomènes provoquent des pertes de qualités organoleptiques des produits, des pertes de matière et entraînent donc des risques significatifs d'intoxication alimentaire.

L'invention a pour objet de pallier ces inconvénients en proposant un procédé de traitement de milieu gazeux contenant des particules contaminantes qui permet de maîtriser efficacement la contamination microbienne aéroportée.

Ce procédé est bien adapté au traitement des milieux gazeux dans les enceintes de conservation de matières vivantes, notamment de produits alimentaires, ce procédé permettant d'améliorer de façon significative leur conservation. Le procédé pourra cependant être appliqué à tout type de milieu gazeux.

Ainsi, l'invention concerne un procédé de traitement de milieu gazeux contenant des particules contaminantes, consistant à :
- générer un flux d'électrons accélérés,
- faire interagir ledit flux d'électrons et ledit milieu gazeux, cette interaction provoquant la rupture ou la destruction desdites particules par ionisation et la stérilisation dudit milieu gazeux.

Pour améliorer l'efficacité de ce procédé de traitement, avant interaction avec le flux d'électrons, le milieu gazeux est accéléré et rendu convergent vers le flux d'électrons selon une veine tourbillonnante.

De préférence, ce procédé consiste également à générer un autre flux d'électrons et à le faire interagir avec le milieu gazeux dont les particules contaminantes ont été préalablement rompues par ionisation, pour provoquer la transformation en gaz desdites particules.

De plus, le procédé selon l'invention consiste avantageusement à faire passer ledit milieu gazeux à travers une matière poreuse active pour provoquer l'absorption de ce milieu gazeux qui pénètre dans les porosités de la matière, puis l'absorption dudit milieu gazeux au cours de laquelle se produit une réaction chimique entre les composés organiques dudit milieu gazeux et la matière elle-même qui transforme les composés organiques volatils en gaz non toxiques, notamment C02 ou S02.

Dans ce cas, le procédé comporte avantageusement une étape de récupération de l'eau présente dans le milieu gazeux, avant passage de celui-ci dans la matière poreuse.

Dans une application préférée, le procédé consiste au préalable à aspirer ledit milieu gazeux depuis une enceinte, telle qu'une cuve de réfrigérateur, puis à refouler, après traitement, ledit milieu gazeux dans cette enceinte.

L'invention concerne également un dispositif de traitement de milieu gazeux contenant des particules contaminantes, ce dispositif comprenant un stérilisateur avec :
- une enveloppe dans laquelle le milieu gazeux est destiné à circuler, comprenant une première ouverture pour l'entrée du milieu gazeux et une deuxième ouverture pour la sortie du milieu gazeux traité et
- une première et une deuxième plaques électriquement conductrices définissant une première chambre dans ladite enveloppe, la première plaque étant fixée au niveau de ladite première ouverture, raccordée au potentiel positif d'une alimentation électrique et perforée de canaux pour accélérer le milieu gazeux, tandis que la deuxième plaque, raccordée au potentiel négatif de ladite alimentation électrique, supporte deux torés concentriques et espacés, placés dans ladite première chambre et destinés à émettre des électrons, le tore interne de plus petit diamètre étant le plus éloigné de la première plaque, dont les canaux comportent un convergeant et un divergeant dont les axes convergent vers le centre du tore interne et étant répartis dans la plaque de façon à créer une turbulence périphérique, de telle sorte que le flux gazeux circule selon une veine tourbillonante qui converge vers le centre du tore interne.

De préférence, le dispositif comporte des ubes s'étendant vers l'intérieur de la première chambre et étant en contact électrique avec la première plaque pour déterminer des cavités de résonance magnétique.

De préférence, la deuxième plaque est perforée de trous traversants et définit, avec une troisième plaque, une deuxième chambre dans ladite enveloppe, la deuxième plaque supportant de plus au moins une électrode s'étendant dans la deuxième chambre et la troisième plaque étant fixée au niveau de la deuxième ouverture de l'enveloppe et supportant au moins un tore s'étendant dans ladite deuxième chambre en direction de ladite électrode, cette troisième plaque étant électriquement conductrice et raccordée au potentiel positif de ladite alimentation électrique et comportant des trous traversants pour évacuer ledit milieu gazeux de ladite enveloppe du stérilisateur.

L'électrode portée par la deuxième plaque est avantageusement sensiblement cylindrique et comporte sur sa périphérie des aubes pour former des cavités de résonance magnétique.

De préférence, le dispositif de traitement selon l'invention comprend au moins un filtre destiné à être traversé par le milieu gazeux, avec un boîtier rempli au moins partiellement d'une matière active poreuse comprenant une substance oxydante, une substance oxydoréductrice et une substance avide d'oxygène, pour la conversion de composés organiques volatils en gaz non toxiques, du type C02 ou S02.

Cette matière active comporte avantageusement environ 47 à 52% en poids d'une substance composite de silicium et de carbone, environ 12 à 20 % en poids de carbone, environ 5 à 7 % en poids d'hydroxyle et environ 1 à 2 % en poids d'oxygène, sa porosité étant notamment comprise entre 65 et 90% en volume.

De préférence, le filtre comporte une plaque en nickel placée à l'intérieur de la matière active poreuse et destinée à être mise sous potentiel électrique, cette plaque comportant des fenêtres revêtues de mousse de platine.

Il est avantageusement prévu, à l'entrée du filtre, des moyens pour récupérer l'eau présente dans le milieu gazeux.

Egalement de préférence, la plaque du filtre comporte des résistances électriques dont le fonctionnement en température peut être programmé pour assurer le recyclage et la régénération de la matière active.

Enfin, l'invention concerne un appareil réfrigéré comportant au moins un compartiment de conservation associé à un dispositif de traitement selon l'invention et à des moyens de réglage de la température, de l'humidité relative et de la ventilation, à des valeurs adaptées aux produits destinés à être placés dans ledit compartiment.

De préférence, le dispositif de traitement associé audit compartiment de conservation comporte deux filtres, le premier filtre étant situé à l'entrée d'un circuit de ventilation aspirant le milieu gazeux contenu dans le compartiment de conservation et le refoulant vers le stérilisateur du dispositif, le deuxième filtre étant situé à la sortie dudit circuit de ventilation et recevant le milieu gazeux traité par ledit stérilisateur pour refouler le milieu gazeux filtré dans le compartiment de conservation.

L'invention sera mieux comprise, et d'autres buts, avantages et caractéristiques de celle-ci apparaîtront plus clairement à la lecture de la description qui suit et qui est faite au regard des dessins annexés qui représentent des exemples non limitatifs de réalisation de l'invention et sur lesquels :
- la figure 1 illustre schématiquement un exemple de dispositif de traitement selon l'invention, comportant deux filtres et un stérilisateur;
- la figure 2 est une vue en coupe axiale d'un exemple de stérilisateur ;
- la figure 3 est une vue en coupe radiale selon III-III de la figure 2 ;
- la figure 4 est une vue en coupe radiale selon IV-IV de la figure 2 ;
- la figure 5 est une vue en coupe radiale selon V-V de la figure 2 ;
- la figure 6 est une vue en perspective avec arrachement partiel d'un exemple de filtre du dispositif illustré à la figure 1;
- la figure 7 est une vue générale en perspective d'un appareil réfrigéré selon l'invention ; et
- la figure 8 est une vue en coupe selon VIII-VIII de la figure 7.

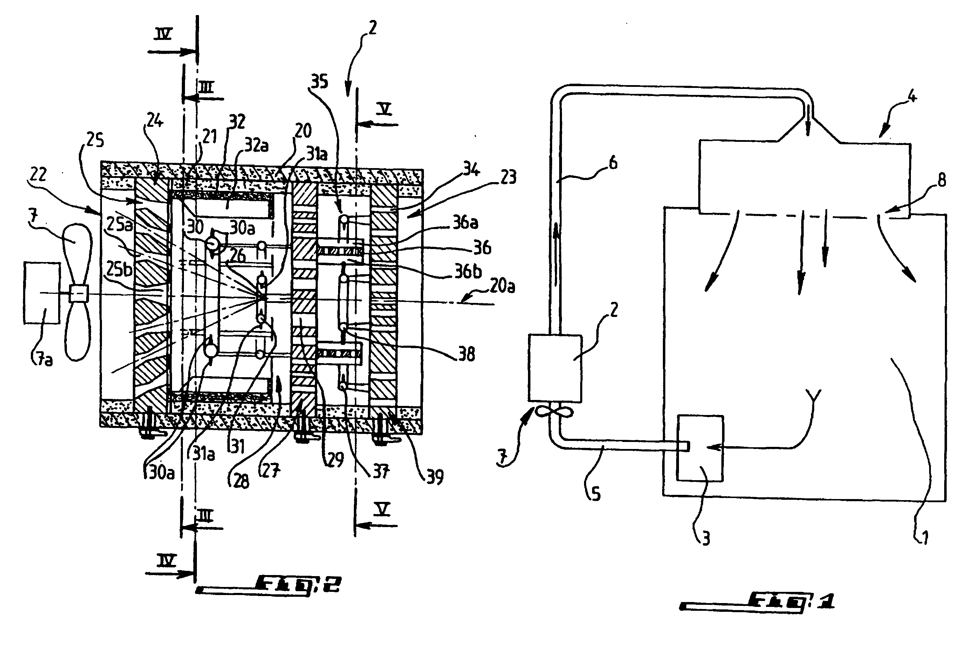

En référence tout d'abord à la figure 1, le dispositif de traitement selon l'invention est associé à une enceinte 1 renfermant un milieu gazeux contenant des particules contaminantes, notamment du type micro-organismes, bactéries, levures, moisissures et virus. Cette enceinte 1 peut notamment être une chambre de conservation d'un réfrigérateur.

Le dispositif selon l'invention illustré à la figure 1 comporte un stérilisateur 2 et deux filtres 3 et 4. L'élément essentiel de ce dispositif de traitement est le stérilisateur 2, les filtres 3 et 4 pouvant être omis.

Les différents éléments constitutifs du dispositif de traitement sont placés sur un circuit de ventilation comprenant deux conduits 5 et 6. De préférence, un ventilateur 7 est associé à ce circuit.

De façon schématique, le milieu gazeux présent dans l'enceinte 1 est aspiré à travers le filtre 3 pour être amené jusqu'au stérilisateur 2, par l'intermédiaire du conduit 5. Le milieu gazeux est ensuite amené sur le filtre 4, par le conduit 6.

Finalement, le milieu gazeux traité par le dispositif selon l'invention est réintroduit dans l'enceinte 1, par exemple par des déflecteurs 8.

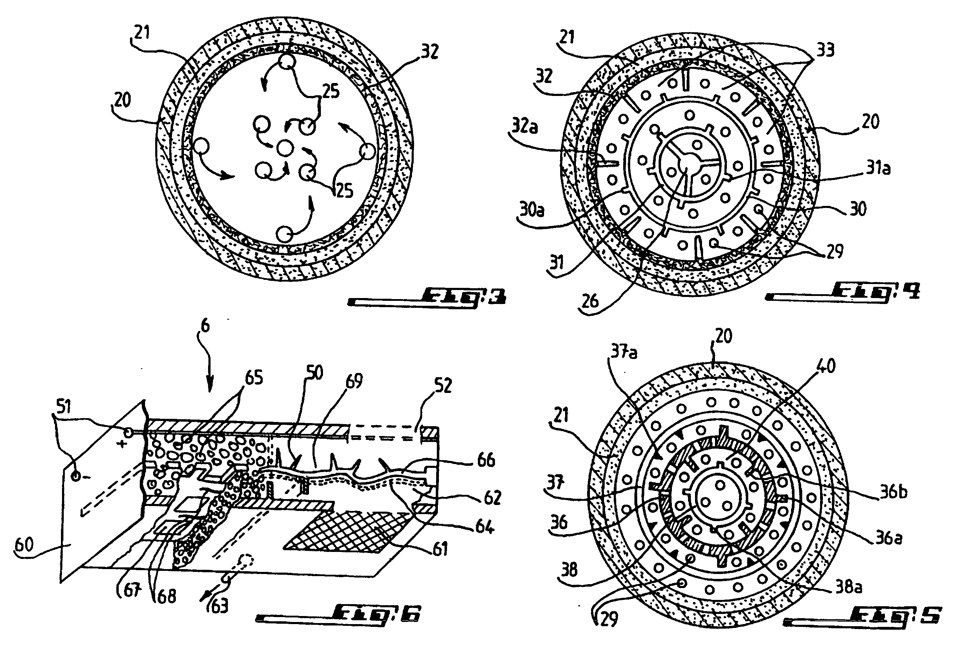

Le stérilisateur 2 sera décrit plus en détail en référence aux figures 2 à 5.

La figure 2 représente un exemple de stérilisateur du dispositif de traitement selon l'invention, en coupe axiale.

Ce stérilisateur comporte une enveloppe 20, ici sensiblement cylindrique, avec une enveloppe interne 21 réalisée en une matière isolante diélectrique, différentes pièces métalliques étant prévues à l'intérieur de l'enceinte 20 pour permettre l'ionisation des particules contaminantes présentes dans le milieu gazeux, destiné à circuler dans le stérilisateur 2.

L'enveloppe 20 comporte une première ouverture 22 pour l'entrée du milieu gazeux dans le stérilisateur 2 et une deuxième ouverture 23 pour la sortie du milieu gazeux, après traitement dans le stérilisateur 2.

Le stérilisateur 2 comporte une première plaque 24, en un matériau électriquement conducteur, qui est raccordée au potentiel positif d'une alimentation électrique.

Cette première plaque 24 est fixée au niveau de la première ouverture 22 et elle comporte des moyens pour accélérer le milieu gazeux entrant dans le stérilisateur 2.

Dans cet exemple de réalisation, ces moyens sont constitués par des canaux 25 traversant la première plaque 24.

Ces canaux sont en forme de tuyère et comportent un convergent 25a et un divergent 25b, dont les axes convergent en un point 26 situé sur l'axe 20a de l'enveloppe et qui constitue un centre électrique.

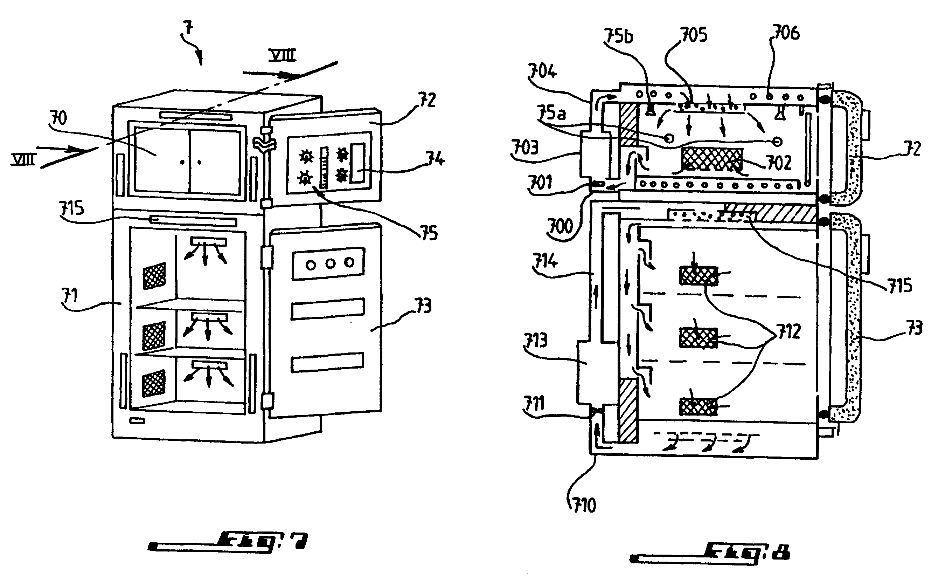

Les canaux 25 ont une forme telle qu'ils accélèrent le flux gazeux pénétrant dans le stérilisateur. De plus, ils sont répartis dans la plaque 24 de façon à créer une turbulence périphérique, de telle sorte que le flux gazeux circule selon une veine tourbillonnante qui converge vers le centre électrique 26. Ceci est illustré à la figure 3, sur laquelle seuls quelques canaux 25 sont représentés.

A l'intérieur de l'enveloppe 20 est également prévue une deuxième plaque 27 qui est disposée, comme la première plaque 24, sensiblement perpendiculairement à l'axe 20a de l'enveloppe 20.

Cette plaque 27 est réalisée en un matériau électriquement conducteur et reliée au potentiel négatif de l'alimentation électrique.

Ainsi, la première et la deuxième plaques 24 et 27 définissent une première chambre 28 dans l'enveloppe 20.

La deuxième plaque 27 est percée de trous traversants 29 qui sont sensiblement parallèles à l'axe 20a de l'enveloppe 20.

Cette deuxième plaque 27 supporte un tore cylindrique externe 30 et un tore cylindrique interne 31.

De préférence, le tore interne 31 est éloigné de la deuxième plaque 27, selon l'axe 20a, d'une distance correspondant sensiblement au tiers de la distance axiale entre la première et la deuxième plaques 24 et 27. De même, le tore externe 30 est, de préférence, situé à une distance axiale de la deuxième plaque 27 correspondant sensiblement aux deux tiers de la distance entre les deux plaques 24 et 27.

Ainsi, les deux tores 30 et 31 s'étendent dans la chambre 28 en étant sensiblement centrés sur l'axe 20a de l'enveloppe 20. Comme indiqué précédemment, l'orientation des tuyères 25 est telle que le cône défini par l'axe des différentes tuyères 25 est centré sur le centre électrique 26 correspondant au centre du tore interne 31.

A l'intérieur de la chambre 28 est également prévue une pièce cylindrique 32 qui est en contact électrique avec la première plaque 24 et, de préférence, en contact avec l'enveloppe interne 21.

Cette pièce cylindrique 32 comporte à sa périphérie intérieure des aubes 32a qui s'étendent à l'intérieur de la première chambre 28. Comme l'illustre la figure 4, ces aubes 32a ont pour fonction de délimiter des cavités de résonance magnétique 33 qui sont centrées sur les deux tores 30 et 31, destinés à émettre des électrons de forte énergie sous l'effet de la mise sous fort potentiel négatif de la deuxième plaque 27.

Les éléments constitutifs du stérilisateur 2 qui viennent d'être décrits constituent les éléments essentiels du stérilisateur, comme le montrera son fonctionnement qui sera décrit ultérieurement.

Cependant, le stérilisateur 2 peut également comprendre, à l'intérieur de l'enveloppe 20, une troisième plaque 39 qui est fixée sensiblement perpendiculairement à l'axe 20a de l'enveloppe et au niveau de la deuxième ouverture 23.

Cette plaque 39 est réalisée en un matériau électriquement conducteur et est reliée au potentiel positif de l'alimentation électrique. Elle est perforée de trous 34 qui s'étendent sensiblement selon l'axe 20a. Ces trous 34 permettent au milieu gazeux traité par le stérilisateur de sortir par la deuxième ouverture 23.

La deuxième et la troisième plaques 27 et 39 définissent une deuxième chambre 35 du stérilisateur 2.

Sur la deuxième plaque 27 est fixée une électrode cylindrique 36 perforée qui s'étend dans la deuxième chambre 35. Comme l'illustre la figure 5, cette électrode 36 comporte sur sa périphérie extérieure des aubes 36a et sur sa périphérie intérieure des aubes 36b qui définissent des cavités de résonance magnétique 40.

Par ailleurs, sur la troisième plaque 39 sont fixés un tore externe 37 et un tore interne 38 qui sont, comme l'électrode 36, sensiblement centrés sur l'axe 20a de l'enveloppe 20. Le tore externe 37 comporte sur sa périphérie intérieure des excroissances 37a, tandis que le tore interne 38 comporte, sur sa périphérie extérieure des excroissances 38a.

Le fonctionnement du stérilisateur 2 est le suivant.

Le stérilisateur 2 reçoit le milieu gazeux à traiter qui circule à l'intérieur du stérilisateur, sous l'impulsion du ventilateur 7 entrainé par un motoréducteur 7a dont la vitesse est asservie, en fonction de la perte de charge existant entre le flux de milieu gazeux au niveau de la première ouverture 22 et au niveau de la deuxième ouverture 23.

De manière avantageuse, le milieu gazeux entrant par la première ouverture 22 du stérilisateur 2 a une vitesse comprise entre 20 et 80 cm/s. En passant sur les tuyères 25, le milieu gazeux pénètre dans la première chambre 28 à une vitesse stabilisée comprise entre 70 et 200 cm/s.

Les tuyères 25 étant orientées sur le centre électrique 26, le milieu gazeux circule selon une veine tourbillonnante qui est traitée par des électrons basse énergie émis par les deux tores 30 et 31 fixés sur la deuxième plaque 29.

L'énergie de ces électrons est avantageusement d'environ 0,1 Mev, l'alimentation électrique du stérilisateur étant réalisée à des potentiels compris entre 20 et 30 10³ volts.

Ainsi, le stérilisateur 2 génère un flux d'électrons accélérés dont l'interaction avec le milieu gazeux chargé de particules contaminantes, telles que des micro-organismes, provoquent l'ionisation des particules, ce qui entraîne des modifications chimiques et des effets biologiques, en détruisant toutes les structures moléculaires des acides nucléiques et plus généralement, tous les constituants des cellules eucayotes, procaryotes et acaryotes ou virus. Cette interaction provoque également la rupture des chaînes et des liaisons d'hydrogène, tout en amenant des oxydations destructrices des structures lypoprotéines des membranes.

Les cavités de résonance magnétiques 33 prévues dans la première chambre 28 permettent l'amplification énergétique de l'émission ionique et sa modularité fréquentielle, c'est-à-dire la génération de flux d'électrons avec une agitation à fréquence variable en liaison avec l'état de l'atmosphère environnante. Grâce à cette caractéristique, le stérilisateur 2 et le procédé selon l'invention permettent de détruire tous les constituants microbiologiques, quelle que soit leur radiosensibilité. Les excroissances 30a et 31a prévues sur les périphéries externe et interne respectivement du tore externe 30 et du tore interne 31 permettent de privilégier la diffusion des électrons émis par les tores, au centre des cavités de résonance magnétique 33.

Le milieu gazeux déjà traité dans la première chambre pénètre ensuite dans la deuxième chambre en passant à travers les trous 29 ménagés dans la deuxième plaque 27 et pénètrent dans la deuxième chambre 35.

Les tores 37 et 38 étant reliés au potentiel positif et la deuxième plaque 27 au potentiel négatif, la destruction et la décohésion moléculaire du reste des particules contenues dans le milieu gazeux circulant dans le stérilisateur sont assurées dans la deuxième chambre 35.

Ainsi, la deuxième plaque 27 constitue une interface entre la première chambre 28 de désintégration des structures biologiques et la deuxième chambre 35 de détérioration des particules déstructurées.

Ces deux chambres permettent la stérilisation ionique du milieu gazeux chargé de micro-organismes qui circule dans le stérilisateur, grâce à l'émission d'électrons accélérés par les tores 30 et 31.

On se réfère maintenant à la figure 6 qui illustrent un filtre tel que ceux représentés à la figure 1 sous les références 3 et 4.

Ce filtre 6 comporte un boîtier 60 qui est destiné à être traversé par un milieu gazeux, pénétrant par une grille 61.

Le milieu gazeux pénétrant dans le filtre 6 peut être relativement humide. C'est pourquoi, il est avantageusement prévu dans le boîtier 60 des moyens pour récupérer l'eau présente dans le milieu gazeux. Dans l'exemple de réalisation illustré à la figure 6, ces moyens sont constitués par une capacité à chicanes 62, l'eau récupérée pouvant être évacuée par la vidange 63 prévue dans le fond du boîtier. La capacité 62 comporte de préférence un revêtement isolant 64.

Le boîtier 60 est partiellement rempli d'une matière active poreuse comprenant une substance oxydante, une substance oxydoréductrice et une substance avide d'oxygène. Cette matière porte la référence 65 sur la figure 6. Elle peut être organisée en granulés ou en plaques.

Cette matière active a pour objet d'épurer le milieu gazeux des composés organiques volatils par filtration oxydoréductrice et oxydation ionique.

A titre d'exemple, une telle matière active poreuse comporte environ 47 à 52 % en poids d'une substance composite de silicium et de carbone, environ 12 à 20 % en poids de carbone, environ 5 à 7 % en poids d'hydroxyle et environ 1 à 2 % en poids d'oxygène, sa porosité étant notamment comprise entre 60 et 85% en volume. La porosité représente le taux d'espace libre dans la matière.

Cette matière active présente une grande surface spécifique grâce à la présence de nombreuses porosités dont les dimensions sont comprises entre 60 et 100 A.

Ces porosités permettent notamment l'adsorption du milieu gazeux qui pénètre dans ces porosités, puis l'absorption de ce milieu gazeux au cours de laquelle se produit une réaction entre les composés organiques volatils du milieu gazeux et la matière elle-même. Cette réaction permet la conversion chimique des composés organiques en gaz non toxiques, notamment du S02 ou C02.

La sensibilité physicochimique de la matière active poreuse peut être accentuée et régulée en humidité relative et en température grâce à une plaque 66 qui peut notamment être placée au milieu de la matière poreuse 65. Cette plaque 66 comporte des fenêtres 67 qui sont revêtues de mousse de platine, la plaque alvéolée étant réalisée en nickel.

La plaque 66 peut être mise sous potentiel électrique de quelques millivolts à quelques volts et elle a pour fonction essentielle de favoriser les conversions chimiques des composés organiques volatils à épurer.

Par ailleurs, comme on le verra dans la suite de la description, cette plaque 66 permet un apport calorifique dosé en fonction de l'humidité relative de l'enceinte dont est extrait le milieu gazeux, et en particulier l'humidité relative des chambres de conservation d'un réfrigérateur.

De plus, la plaque 66 comporte de préférence des résistances électriques 68. Leur fonctionnement en température peut être programmé manuellement ou automatiquement pour assurer le recyclage et la régénération de la matière active 65.

Le milieu gazeux traité par la matière active arrive ensuite dans la chambre 69 et circule sur les électrodes 50 dont la charge électrostatique à fort potentiel est alimentée par les bornes électriques 51.

Le milieu gazeux ressort enfin du filtre 6 par la grille 52.

Ainsi, le milieu gazeux, après passage dans le filtre selon l'invention, est épuré des composés organiques volatils qu'il pouvait contenir. La conversion de ces composés organiques conduit à l'émission de gaz tels que du S02 ou C02 qui sont généralement inhibiteurs pour le développement des micro-organismes, ce qui contribue à l'efficacité du procédé selon l'invention.

Comme cela a été indiqué précédemment, le dispositif selon l'invention comprend essentiellement le stérilisateur 2. Cependant, la présence d'un filtre au niveau de l'aspiration du milieu gazeux depuis l'enceinte 1 et au niveau de la réintroduction du milieu gazeux dans l'enceinte, tel que les filtres 3 et 4 représentés à la figure 1, permet de détruire les composés organiques voltatils ainsi que les odeurs dégagées par le milieu gazeux et notamment dues à la présente d'aldéhydes. Par contre, la destruction des particules contaminantes est essentiellement obtenue grâce au stérilisateur 2.

Le procédé et le dispositif selon l'invention trouvent notamment application dans le traitement des milieux gazeux présents dans les cuves des réfrigérateurs.

Ainsi, l'invention concerne également un appareil réfrigéré comportant au moins une chambre de conservation associée à un dispositif de traitement selon l'invention.

Cet appareil réfrigéré peut également comporter au moins deux chambres de conservation, dont la température et l'humidité relative seront adaptées en fonction des conditions de conservation des produits alimentaires placés dans chacune de ces chambres. Un tel appareil réfrigéré permet donc un traitement différencié des chambres de conservation, tout en garantissant la stérilisation et la filtration des milieux gazeux contenus dans chacune de ces chambres spécialisées.

On se réfère aux figures 7 et 8 qui illustrent un appareil réfrigéré selon l'invention.

Ce réfrigérateur 7b comporte dans cet exemple deux compartiments de conservation 70 et 71, chacun d'eux étant fermé par une porte 72, respectivement 73.

Chacun de ces compartiments 70 et 71 comporte des moyens pour adapter le milieu gazeux qu'il contient aux conditions de température, d'humidité relative et de ventilation convenant au type de matières conservées.

Ainsi, dans cet exemple, la température du milieu gazeux contenue dans le compartiment 71 sera régulée entre 3 et 7°C.

Le compartiment 70 est, dans cet exemple, destiné à conserver des viandes et des poissons.

La température de ce compartiment 70 sera réglée à environ 0°C par le moyen de réglage en température 74, tandis que l'humidité relative sera réglée entre 40 et 80 % grâce à un dispositif 75 qui comporte notamment au moins une sonde 75a de mesure d'humidité et au moins une buse 75b pour l'injection d'eau pulvérisée dans le compartiment 70.

Comme le montre également la figure 8, dans chacun des compartiments réfrigérés 70, respectivement 71, le milieu gazeux présent dans le compartiment est aspiré par le circuit de ventilation qui comprend un premier conduit 700, respectivement 710, l'aspiration étant obtenue grâce à un ventilateur 701, respectivement 711.

Dans chaque compartiment, l'air est aspiré au travers d'au moins un filtre 702, respectivement 712. Les filtres sont de préférence placés sur une paroi latérale, l'aspiration étant bien sûr également effectuée sur cette paroi latérale.

Les filtres 702 et 712 présentent les caractéristiques qui ont été décrites pour le filtre 6, en référence à la figure 6.

Ainsi, le milieu gazeux qui circule dans le premier conduit 700, 710 est déjà épuré d'au moins une partie des composés organiques volatils qu'il pouvait contenir.

Ce milieu gazeux traverse ensuite le stérilisateur 703, respectivement 713 pour traiter l'essentiel des particules contaminantes du milieu gazeux. De préférence, le stérilisateur est mis sous une tension de 20000 volts.

Les essais réalisés ont mis en évidence que, grâce à cette stérilisation, les germes profonds anaérobiques sont bloqués et les germes psychrotrophes responsables de l'altération superficielle sont très ralentis. Cette stérilisation qui détruit pratiquement tous les micro-organismes présents dans le milieu gazeux permet ainsi de conserver les produits présents dans chacun des compartiments avec une qualité optimale.

L'air qui sort de chaque stérilisateur 703, respectivement 713 est conduit par un deuxième conduit 704, respectivement 714 sur un deuxième filtre 705, 715 qui est de préférence placé à la partie supérieure de chaque compartiment 70, respectivement 71. Ces deuxièmes filtres permettent d'épurer encore le milieu gazeux traité par le stérilisateur, notamment pour convertir les composés organiques volatils et absorber les odeurs, avant de refouler le milieu gazeux traité dans chaque compartiment.

Dans l'exemple de réalisation illustré à la figure 8, le compartiment de conservation 70 comporte un circuit de réfrigération complémentaire 706 pour refroidir davantage le milieu gazeux qui est réintroduit dans le compartiment 70 à travers le filtre 705.

Le procédé et le dispositif selon l'invention peuvent trouver de nombreuses applications, différentes du traitement d'atmosphères de conservation, et peuvent notamment être utilisés pour traiter de l'air circulant dans des dispositifs de climatisation.

Les signes de référence insérés après les caractéristiques techniques figurant dans les revendications ont pour seul but de faciliter la compréhension de ces dernières et ne sauraient en limiter la portée.

## Revendications

1. Procédé de traitement de milieu gazeux contenant des particules contaminantes, notamment des structures biologiques polluantes aéroportées, ce procédé consistant à :
générer un flux d'électrons accélérés,
- faire interagir ledit flux d'électrons et ledit milieu gazeux, cette interaction provoquant la rupture ou la destruction desdites particules par ionisation et la stérilisation dudit milieu gazeux, et
- avant interaction avec le flux d'électrons, à accélérer ledit milieu gazeux et à le rendre convergent vers le flux d'électrons selon une veine tourbillonnante.

2. Procédé selon la revendication 1 consistant à générer un autre flux d'électrons et à le faire interagir avec le milieu gazeux dont les particules contaminantes ont été préalablement rompues par ionisation, pour provoquer la transformation en gaz desdites particules.

3. Procédé selon l'une des revendications 1 ou 2, consistant à faire passer ledit milieu gazeux à travers une matière poreuse active pour provoquer l'adsorption de ce milieu gazeux qui pénètre dans les porosités de la matière, puis l'absorption dudit milieu gazeux, au cours de laquelle se produit une réaction chimique entre les composés organiques dudit milieu gazeux et la matière elle-même qui transforme les composés organiques volatils en gaz non toxiques, notamment CO2 ou SO2.

4. Procédé selon la revendication 3, consistant à récupérer l'eau présente dans le milieu gazeux avant le passage du milieu gazeux dans la matière poreuse.

5. Procédé selon l'une des revendications 1 à 4, consistant au préalable à aspirer ledit milieu gazeux depuis une enceinte, telle qu'une cuve de réfrigérateur, puis à refouler, après traitement, ledit milieu gazeux dans cette enceinte.

6. Dispositif de traitement de milieu gazeux contenant des particules contaminantes, ce dispositif comprenant un stérilisateur (2)avec :
- une enveloppe (20) dans laquelle le milieu gazeux est destiné à circuler, comprenant une première ouverture (22) pour l'entrée du milieux gazeux et une deuxième ouverture (23) pour la sortie du milieu gazeux traité et
- une première et une deuxième plaques (24, 27) électriquement conductrices définissant une première chambre (28) dans ladite enveloppe, la première plaque (24) étant fixée au niveau de ladite première ouverture, raccordée au potentiel positif d'une alimentation électrique et perforée de canaux (25) pour accélérer le milieu gazeux, tandis que la deuxième plaque (27), raccordée au potentiel négatif de ladite alimentation électrique, supporte deux tores (30, 31) concentriques et espacés, placés dans ladite première chambre (28) et destinés à émettre des électrons, le tore interne (31) de plus petit diamètre étant le plus éloigné de la première plaque (24), dont les canaux (25) comportent un convergeant (25a) et un divergeant (25b) dont les axes convergent vers le centre (26) du tore interne (31) et étant répartis dans la plaque (24) de façon à créer une turbulence périphérique, de telle sorte que le flux gazeux circule selon une veine tourbillonante qui converge vers le centre (26) du tore interne (31).

7. Dispositif selon la revendication 6, comprenant des aubes (32a) s'étendant vers l'intérieur de la première chambre (28) et étant en contact électrique avec la première plaque (24) pour déterminer des cavités de résonance magnétique (33).

8. Dispositif selon la revendication 6 où 7, dans lequel la deuxième plaque (27) est perforée de trous traversants (29) et définit, avec une troisième plaque (39), une deuxième chambre (35) dans ladite enveloppe, la deuxième plaque (39) supportant au moins une électrode (36) (23) de l'enveloppe et supportant au moins un tore (37, 38) s'étendant dans ladite deuxième chambre (35) en direction de ladite electrode (36), cette troisième plaque (39) étant électriquement conductrice et raccordée au potentiel positif de ladite alimentation électrique et comportant des trous traversants (34) pour évacuer ledit milieu gazeux de ladite enveloppe (20) du stérilisateur (2).

9. Dispositif selon la revendication 8, dans lequel l'électrode (36) portée par la deuxième plaque (27) est avantageusement sensiblement cylindrique et comporte sur sa périphérie des aubes (36a, 36b), pour former des cavités de résonance magnétique (40).

10. Dispositif selon l'une des revendications 6 à 9, comprenant au moins un filtre (6) destiné à être traversé par le milieu gazeux, avec un boîtier (50) rempli au moins partiellement d'une matière active poreuse (65) comprenant une substance avide oxydante, une substance oxydoréductrice et une substance d'oxygène, pour la conversion de composés organiques volatils en gaz non toxique, du type CO2 ou SO2.

11. Dispositif selon la revendication 10, dans lequel la matière active (65) présente dans le filtre (6) comporte environ 47 à 52% en poids d'une substance composite de silicium et de carbone, environ 12 à 20% en poids de carbone, environ 5 à 7% en poids d'hydroxyle et environ 1 à 2 % en poids d'oxygène, sa porosité étant notamment comprise entre 60 et 85% en volume.

12. Dispositif selon la revendication 10 ou 11, dans lequel le filtre (6) comporte une plaque (66) en nickel placée à l'intérieur de la matière active poreuse (65) et destinée à être mise sous potentiel électrique, cette plaque comportant des fenêtres (67) revêtues de mousse de platine.

13. Dispositif selon l'une des revendications 10 à 12, dans lequel sont prévus, à l'entrée du filtre, des moyens (62) pour récupérer l'eau présente dans le milieu gazeux.

14. Dispositif selon l'une des revendications 10 à 13, dans lequel la plaque (50) comporte des résistances électriques dont le fonctionnement en température peut être programmé pour assurer le recyclage et la régénération de la matière active.

15. Appareil réfrigéré (7b) comportant au moins un compartiment de conservation (70, 71) associé à un dispositif de traitement (702, 701, 705, 712, 713, 715) selon l'une des revendications 6 à 14 et à des moyens de réglage ( 74, 75) de la température, de l'humidité relative et de la ventilation, à des valeurs adaptées aux produits destinés à être placés dans ledit compartiment.

16. Appareil selon la revendication 15, dans lequel le dispositif de traitement associé audit compartiment (70, 71) comporte deux filtres (702, 705 ; 712, 715), le premier filtre (702 ; 712) étant situé à l'entrée d'un circuit de ventilation (700, 704 ; 710, 714) aspirant le milieu gazeux contenu dans le compartiment de conservation, et le refoulant vers le stérilisateur (701 ; 711) du dispositif, le deuxième filtre (705; 715) étant situé à la sortie dudit circuit de ventilation et recevant le milieu gazeux traité par le stérilisateur pour refouler le milieu gazeux filtré dans le compartiment de conservation (70, 71).

## Patentansprüche

1. Verfahren zum Behandeln von gasförmigen Medien, die kontaminierende Teilchen enthalten, insbesondere durch die Luft beförderte verunreinigende biologische Strukturen, wobei dieses Verfahren darin besteht:
- einen Fluss von beschleunigten Elektronen zu erzeugen,
- den besagten Elektronenfluss und das besagte gasförmige Medium in Wechselwirkung zu bringen, wobei die besagten Teilchen durch Ionisierung und die Sterilisierung des besagten gasförmigen Mediums gebrochen oder zerstört werden und
- das besagte gasförmige Medium vor der Wechselwirkung mit dem Elektronenfluss zu beschleunigen und es gemäß einem wirbelnden Strahl zum Elektronenfluss zusammenlaufen zu lassen.

2. Verfahren gemäß Anspruch 1, das darin besteht, einen weiteren Elektronenfluss zu erzeugen und ihn in Wechselwirkung mit dem gasförmigen Medium zu bringen, dessen kontaminierende Teilchen vorangehend durch Ionisierung gebrochen wurden, um die Umsetzung der besagten Teilchen in Gas zu verursachen.

3. Verfahren gemäß Anspruch 1 oder 2, das darin besteht, das besagte gasförmige Medium durch einen aktiven porösen Stoff zu führen, um die Absorption des gasförmigen Mediums, welches in die Porositäten des Stoffs eindringt, zu verursachen, und dann die Absorption des besagten gasförmigen Mediums, im Laufe welcher eine chemische Reaktion zwischen den organischen Verbindungen des besagten gasförmigen Mediums und dem Stoff selber stattfindet, welche die flüchtige organische Verbindung in ein nicht-giftiges Gas, nämlich CO₂ oder SO₂ umsetzt.

4. Verfahren gemäß Anspruch 3, das darin besteht, das im gasförmigen Medium anwesende Wasser zurückzugewinnen, bevor das gasförmige Medium in den porösen Stoff geführt wird.

5. Verfahren gemäß einer der Ansprüche 1 bis 4, das darin besteht, im vorhinein das besagte gasförmige Medium aus einem abgeschlossenen Bereich wie einen Kühlschrankbehälter abzusaugen und das besagte gasförmige Medium nach der Behandlung in den besagten abgeschlossenen Bereich zurückzuführen.

6. Vorrichtung zum Behandeln von kontaminierenden Teilchen enthaltenden gasförmigen Medien, wobei diese Vorrichtung aus einem Sterilisator (2) besteht mit:
- einer Hülle (20), in der das gasförmige Medium zirkulieren soll, welche aus einer ersten Öffnung (22) zum Eintreten des gasförmigen Mediums und einer zweiten Öffnung (23) zum Austreten des behandelten gasförmigen Mediums besteht, und mit
- einer ersten und einer zweiten elektrisch leitenden Platte (24, 27), die eine erste Kammer (28) in der besagten Hülle bestimmen, wobei die erste Platte (24) in der Höhe der ersten Öffnung befestigt ist und an das positive Potential einer Elektrizitätsversorgung angeschlossen ist und mit Kanälen (25) zur Beschleunigung des gasförmigen Mediums durchbohrt ist, während die zweite Platte (27), die an das negative Potential der besagten Elektrizitätsversorgung angeschlossen ist, zwei konzentrische und getrennte Ringkerne (30, 31) unterstützt, die sich in der ersten Kammer befinden (28) und dazu bestimmt sind, Elektronen zu senden,
wobei der innere Ringkern (31) mit einem kleineren Durchmesser weiter von der ersten Platte (24) entfernt ist, deren Kanäle (25) beziehungsweise zusammenlaufend (25a) und auseinanderlaufend (25b) sind, wobei ihre Achsen zur Mitte (26) des inneren Ringkerns zusammenlaufen (31), wobei sie so auf der Platte (24) verteilt sind, dass eine solche periphere Turbulenz entsteht, dass der Gasfluss gemäß einem wirbelnden Strahl, der zur Mitte (26) des inneren Ringkerns (31) zusammenläuft, zirkuliert.

7. Vorrichtung gemäß Anspruch 6, die Schaufeln (32a) enthält, die sich in das Innere der ersten Kammer hin (28) erstrecken und in elektrischem Kontakt mit der ersten Platte (24) zur Bestimmung des magnetischen Hohlraumresonators (33) sind.

8. Vorrichtung gemäß dem Anspruch 6 oder 7, in welcher die zweite Platte (27) mit Löchern (29) durchbohrt ist und welche zusammen mit einer dritten Platte (39) eine zweite Kammer (35) in der besagten Hülle bestimmt, wobei die zweite Platte (39) mindestens eine Elektrode (36) (23) der Hülle und mindestens einen Ringkern (37, 38), der sich in der zweiten Kammer (35) zur besagten Elektrode hin erstreckt (36), aufweist, wobei die dritte Platte (39) elektrisch leitend ist und an das positive Potential der besagten Elektrizitätsversorgung angeschlossen ist und mit Löchern durchbohrt ist (34) um das besagte gasförmige Medium aus der besagten Hülle (20) des Sterilisators (2) abzuführen.

9. Vorrichtung gemäß Anspruch 8, in welcher die auf der zweiten Platte (27) ruhende Elektrode (36) vorteilhaft wesentlich zylinderförmig ausgebildet ist und and ihrem-Rand Schaufeln (36a, 36b) aufweist, um die magnetischen Hohlraumresonatoren (40) zu bilden.

10. Vorrichtung gemäß einer der Ansprüche 6 bis 9, die aus mindestens einem Filter (6) besteht, der dazu bestimmt ist, vom gasförmigen Medium durchdrungen zu werden, mit einem Gehäuse (60), das mindestens zu Hälfte mit einem aktiven porösen Stoff (65) gefüllt ist, der eine Oxydations-anziehende Substanz, eine Oxydations-reduzierende Substanz und eine Sauerstoffsubstanz aufweist, um die flüchtige organische Verbindung in ein nicht-giftiges Gas wie CO₂ oder SO₂ umzusetzen.

11. Vorrichtung gemäß Anspruch 10, in welcher der sich im Filter (6) befindende aktive Stoff (65) zu circa 47 bis 52% seines Gewichtes aus einer Siliziumund Kohlenverbindungssubstanz, zu circa 17 bis 20% seines Gewichts aus Kohle, zu circa 5 bis 7% seines Gewichts aus Hydroxyl und zu circa 1 bis 2% seines Gewichts aus Sauerstoff besteht, wobei die Porosität zwischen 60% und 85% des Volumens ausmacht.

12. Vorrichtung gemäß dem Anspruch 10 oder 11, in welcher der Filter (6) eine Platte (66) aus Nickel aufweist, die innerhalb des aktiven porösen Stoffs (65) angeordnet ist und dazu bestimmt ist, unter elektrisches Potential gesetzt zu werden, wobei diese Platte mit Platinschaum bedeckte Fenster (67) aufweist.

13. Vorrichtung gemäß einer der Ansprüche 10 bis 12, in welcher am Eingang des Filters Mittel (62) zur Aufnahme des sich im gasförmigen Medium befindenden Wassers vorgesehen sind.

14. Vorrichtung gemäß einer der Ansprüche 10 bis 13, in welcher die Platte (50) elektrische Widerstände aufweist, deren Betrieb der Temperatur hinsichtlich programmiert werden kann, um die Wiederverwertung und Wiedergewinnung des aktiven Stoffs zu gewährleisten.

15. Gekühlter Apparat (7b), welcher mindestens ein Aufbewahrungsfach (70, 71), das einer Behandlungsvorrichtung (702 ; 701, 705, 712, 713, 715) gemäß einer der Ansprüche 6 bis 14 zugeordnet ist, so wie den Mitteln zur Einstellung (74, 75) der Temperatur, der relativen Luftfeuchtigkeit und der Belüftung auf Werte entsprechend den Produkten, die in dem besagten Fach aufbewahrt werden sollen.

16. Apparat gemäß Anspruch 15, wobei die dem besagten Fach (70, 71) zugeordnete Behandlungsvorrichtung zwei Filter (702, 705 ; 712, 715) aufweist, wobei der erste Filter (702 ; 712) sich am Eingang des Belüftungskreislaufs (700, 704 ; 710, 714) befindet, welcher das im Aufbewahrungsfach enthaltene gasförmige Medium aufsaugt, und es zum Sterilisator (701 ; 711) der Vorrichtung hin abführt, und wobei der zweite Filter (705 ; 715) sich am Ausgang des besagten Belüftungskreislaufs befindet und das vom Sterilisator behandelte gasförmige Medium aufnimmt, um das gefilterte gasförmige Medium in das Aufbewahrungsfach (70 ; 71) zurückzuführen.

## Claims

1. Treatment method for gaseous mediums containing contaminating particles, in particular of airborne biological structure, this method consisting of:
- generating a flux of accelerated electrons,
- creating interaction between said electron flux and said gaseous medium, this interaction provoking the rupture or the destruction of the said particles through the ionisation and the sterilisation of said gaseous medium, and
- to accelerate said gaseous medium and to make it converge towards the electron flux through a vortex vein before the interaction with the electron flux.

2. Method according to claim 1 consisting of generating another electron flux and creating interaction with the gaseous medium in which the contaminating particles have been previously broken up though ionisation to provoke the transformation of said particles.

3. Method according to claims 1 or 2, consisting of passing said gaseous medium through an active pcrous material to provoke the adsorption of this gaseous medium that penetrates the porosity of the material, then the absorption of said gaseous medium during which a chemical reaction takes place between the organic components of said gaseous medium and the material itself that transforms the volatile organic components into non-toxic gases, namely CO₂ or SO₂.

4. Method according to claim 3, consisting of recovering the water present in the gaseous medium before the passage of the gaseous medium through the porous material.

5. Method according to any one of claims 1 to 4, consisting of previously sucking the said gaseous medium from a container, such as a cooling tank, and then, after treatment, returning the said gaseous medium to this tank.

6. Treatment device for the gaseous medium containing contaminating particles, this device consisting of: a sterilizer (2) with:
- an envelope (20) destined for the circulation of the gaseous medium, composed of a first opening (22) for the entry of the gaseous medium and a second opening (23) for the exit of the treated gaseous medium, and
- a first and a second electrical conductor plate (24, 27) that compose a first chamber (28) in said envelope, the first plate (24) being fixed at the level of the first opening, connected to the positive potential of an electrical supply and perforated with channels (25) to accelerate the gaseous medium, while the second plate (27) connected to the negative potential of said electrical supply, bears two separately spaced concentric cores (30, 31) positioned in said first chamber (28) and destined for the emission of electrons. The internal core (31) with the smaller diameter is the furthest in distance from the first plate (24) whose channels (25) are composed of a convergent (25a) and a divergent (25b) whose axes converge towards the centre (26) of the internal core (31) and they are arranged in the plate (24) to create peripheral turbulence so that the gaseous flux circulates in a vortex vein that converges towards the centre (26) of the internal core (31).

7. Device according to claim 6 also composed of vanes (32a) arranged towards the interior of the first chamber (28) and in electrical contact with the first plate (24) to determine magnetic resonance cavities (33).

8. Device according to claims 6 or 7, wherein the second plate (27) is perforated with holes that transverse the plate (29), and with a third plate (39), and a second chamber (35) in said envelope, it defines the second plate (39) bearing at least one electrode (36) (23) of the envelope and bearing at least one core (37, 38) and arranged in the said second chamber (35) in the direction of said electrode (36), this third plate (39) being an electric conductor and connected to the positive potential of the said electrical supply and being perforated with the holes (34) to evacuate said gaseous medium from said envelope (20) cf the sterilizer (2).

9. Device according to claim 8, wherein the electrode (36) borne by the second plate (27) is advantageously mainly cylindrical in shape, and bears vanes around its perimeter (36a, 36b) to form magnetic resonance cavities (40).

10. Device according to one of claims 6 to 9, composed of at least one filter (6) destined to be crossed by the gaseous medium, with a box (60) at least partially filled with an active porous material (65) composed of an oxygen absorbent substance, an oxygen reducer substance, and an oxygen substance, for converting the volatile organic mixture into a non-toxic gas such as CO₂ or SO₂.

11. Device according to claim 10, wherein the active material (65) present in the filter (6) is composed of approximately 47 to 52% by weight of a substance composed of silicon and carbon, approximately 12 to 20% by weight of carbon, approximately 5 to 7% by weight of hydroxyl, and approximately 1 to 2% by weight of oxygen, this porosity being composed of between 60 and 85% by volume.

12. Device according to claims 10 or 11, wherein the filter (6) is composed of a nickel plate (66) placed inside the active porous material (65) and destined to be subject to electric potential, this plate containing windows (67) coated with platinum foam.

13. Device according to one of claims 10 to 12, wherein certain means (62) are positioned at the filter entrance to recover the water present in the gaseous medium.

14. Device according to one of claims 10 to 13, wherein the plate (50) is composed of electrical elements whose temperature can be programmed to ensure recycling and regeneration of the active material.

15. Cooling system (7b) composed of at least one conservation compartment (70, 71) associated with a treatment device (702, 701, 705, 712, 713, 715) according to claims 6 to 14, and with a means of control (74, 75) for the temperature, relative humidity, and ventilation according to the values applied to the products destined to be placed in said compartment.

16. System according to claim 15, wherein the treatment device associated with said compartment (70, 71) is composed of two filters (702, 705, 712, 715), the first filter (702, 712) being positioned at the entrance to the ventilation circuit (700, 704; 710, 714) sucking in the gaseous medium contained in the conservation compartment, and returning it towards the sterilizer (701, 711) of the device, the second filter (705, 715) being positioned at the exit of said ventilation circuit, receiving the gaseous medium treated by the sterilizer, and returning it to the conservation compartment (70, 71).
